# EUROPEAN PATENT APPLICATION

(11) **EP 1 527 788 A1**
(43) Date of publication of application: **04.05.2005**
(21) Application number: 04256710.7
(22) Date of filing: 29.10.2004
(51) Int. Cl.: A61L 2/26, A61B 19/02

(54) **Sterilization tray and mat**

(30) Priority: 31.10.2003 US 699274
(71) Applicant: ETHICON, INC., Somerville, NJ 08876 (US)
(72) Inventor: Wi, Su-Syin, Irvine, CA 92614 (US)
(74) Representative: Fisher, Adrian John

(57) **Abstract**

A sterilization container system includes an enclosure having a flexible elastomeric mat therein.

The mat is preferably formed of medical grade silicon.

A pattern of ridges is formed on a lower surface of the mat such that no fold line can traverse the mat without intersecting one or more ridges.

## Description

### BACKGROUND OF INVENTION

This application relates to sterilization trays having instrument holding mats, and more particularly to such trays in which a lower surface of the mat is textured.

Sterilization trays are commonly used for holding instruments during a sterilization procedure. Such trays may comprise a simple box having perforations which is then wrapped and sealed with a semi-permeable wrapping material such as central supply room (CSR) wrap, typically formed of non-woven polypropylene, which is permeable to sterilizing gases yet impermeable to potentially contaminating microorganisms. Sometimes, rather than wrapping the tray, the devices therein are individually packaged. Such a tray or container may take a more complex form in which rather than being wrapped in CSR wrap, a semi-permeable filter material covers one or more apertures into the container and it is otherwise sealed. Examples of such trays and containers are disclosed in U.S. Patent Nos. 6,379,631, 6,572,819 and 4,716,025, each incorporated herein by reference.

Typically, some means for holding instruments against movement within the tray is provided. One of the most popular is a simple flexible mat which is placed inside the tray and which carries upwardly projecting members which restrain lateral movement of the instrument or instruments within the tray. Typically such mats are formed of some form of elastomeric material, of which silicone, such as polydimethylsiloxanes or polymethylphenyl siloxane or fluorinated polydimethylsiloxanes or fluorinated polymethylphenyl siloxane, is popular due to its ability to withstand the heat of steam sterilization, its resistance to absorption of, adsorption of, or degradation from common chemical sterilant vapors such as hydrogen peroxide and ethylene oxide. In some instances, both mats and some other form of instrument holder may be placed into the same tray.

Despite their popularity and convenience, such mats suffer from creep within the trays. They can slide across the tray and fold up at the edges, thus leaving a portion of the tray unprotected and potentially allowing instruments to rattle about within the tray and become damaged during a sterilization procedure and or the handling of the tray thereafter. The problem is exacerbated by the surface texture of the silicone which is preferably smooth to almost polished so as to minimize surface absorption and adsorption. Emery finish 240/360 is preferred.

Frieze et al., in U.S. Patent No. 5,766,561 which is incorporated herein by reference, provide anchors on a bottom surface of a mat which engage drainage holes in the tray. This system requires matched trays and mats and further requires user intervention to place the anchors into the drainage holes. It further limits drainage by blocking some of the holes and may create occlusions at the anchor hole interface which trap liquid. Other protrusions on a mat bottom, such as on the rounded feet on the bottom of the mat disclosed by Brooks, Jr. in U.S. Patent No. 5,098,676 which is incorporated herein by reference, are provided merely to elevate the mat off of the tray bottom surface to prevent liquid from becoming trapped between the mat and the tray.

The present invention improves upon such mats to overcome these and other limitations of the prior trays.

### SUMMARY OF THE INVENTION

A sterilization container system according to the present invention comprises an enclosure defining an interior volume and an upwardly facing surface in the interior volume. A flexible elastomeric mat having a downwardly facing surface rests upon the upwardly facing surface. The downwardly facing surface is provided with a pattern of ridges such that no fold line can traverse the mat from one side to the other without intersecting at least one ridge.

Preferably, no fold line can traverse the mat from one side to the other without intersecting a plurality of the ridges.

Preferably, the ridges have a height of 0.5 mm to 10.0 mm, more preferably a height of 0.75 mm to 5.0 mm, and most preferably a height of 1.0 mm to 4.0 mm.

Preferably, the flexible elastomeric mat is formed of silicone, preferably with a hardness of less than 90A on the Shore A Scale.

Preferably, the pattern extends substantially across a dimension of the lower surface.

The mat can further comprise a plurality of upwardly projecting members and a plurality of apertures therethrough.

The pattern can be continuous or discontinuous. It can comprise concentric shapes. Preferably, it comprises a regular pattern which covers substantially the entire lower surface of the mat.

A method of sterilizing an item according to the present invention comprises the steps of: placing a flexible, elastomeric mat into an enclosure having an interior volume; placing the item onto the mat; and levitating the mat above a bottom surface of the enclosure via a pattern of ridges on a lower surface of the mat, the pattern being such that no fold line can traverse the mat from one side to the other without intersecting at least one ridge.

### BRIEF DESCRIPTION OF THE DRAWINGS

FIG. 1 is an exploded perspective view of a sterilization container according to the present invention;
FIG. 2 is a perspective view of one portion of the mat of FIG. 1;
FIG. 3 is a bottom plan view of a portion of a lower surface of the mat of FIG. 1 showing a pattern of ridges thereon;
FIG. 4 is bottom plan view of a portion of a lower surface of the mat of FIG. 1 showing an alternate pattern of ridges thereon;
FIG. 5 is bottom plan view of a lower surface of the mat of FIG. 1 showing an alternate pattern of ridges thereon;
FIG. 6 is bottom plan view of a lower surface of the mat of FIG. 1 showing an alternate pattern of ridges thereon;
FIG. 7 is bottom plan view of a lower surface of the mat of FIG. 1 showing an alternate pattern of ridges thereon; and
FIG. 8 is bottom plan view of a lower surface of the mat of FIG. 1 showing an alternate pattern of ridges thereon.

### DETAILED DESCRIPTION

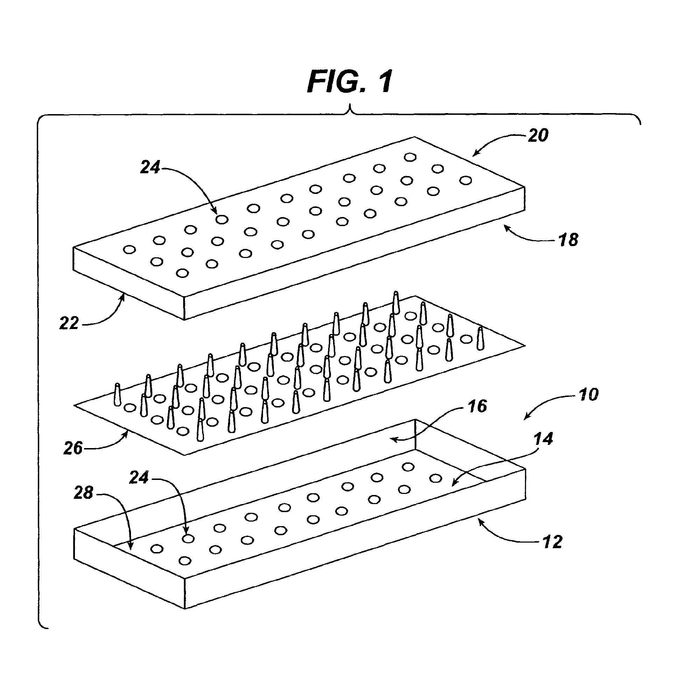

FIG. 1 illustrates a sterilization tray 10 according to the present invention. It comprises a tray base 12 having a bottom wall 14 with upwardly extending sidewalls 16. It also has a lid 18 ( although such a lid 18 is optional) having an upper wall 20 with downwardly depending sidewalls 22 therefrom. Apertures 24 penetrate the upper wall 20 and bottom wall 14 to allow penetration of sterilizing vapors.

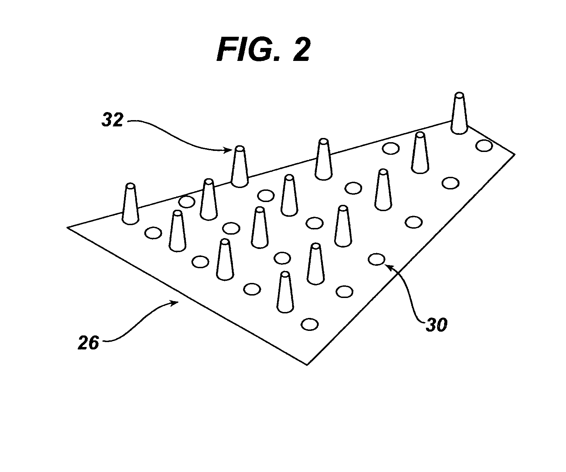

A mat 26 rests upon an upper surface 28 of the bottom wall 14. Turning also to FIG. 2, the mat 26 has a plurality of apertures 32 therethrough and upwardly ascending projections 34 therefrom. Preferably, the mat is formed of a medical grade silicone or some other medically suitable, flexible elastomer or plastic, having sufficient heat resistance capabilities to withstand the heat of a steam sterilization cycle, and which is inert to chemical sterilants, and sufficiently nonabsorbent to chemical sterilants, such as hydrogen peroxide and ethylene oxide, so as to not interfere with the sterilization process. The style, arrangement and number of the apertures 30 and projections 32 can be varied by those of skill in the art.

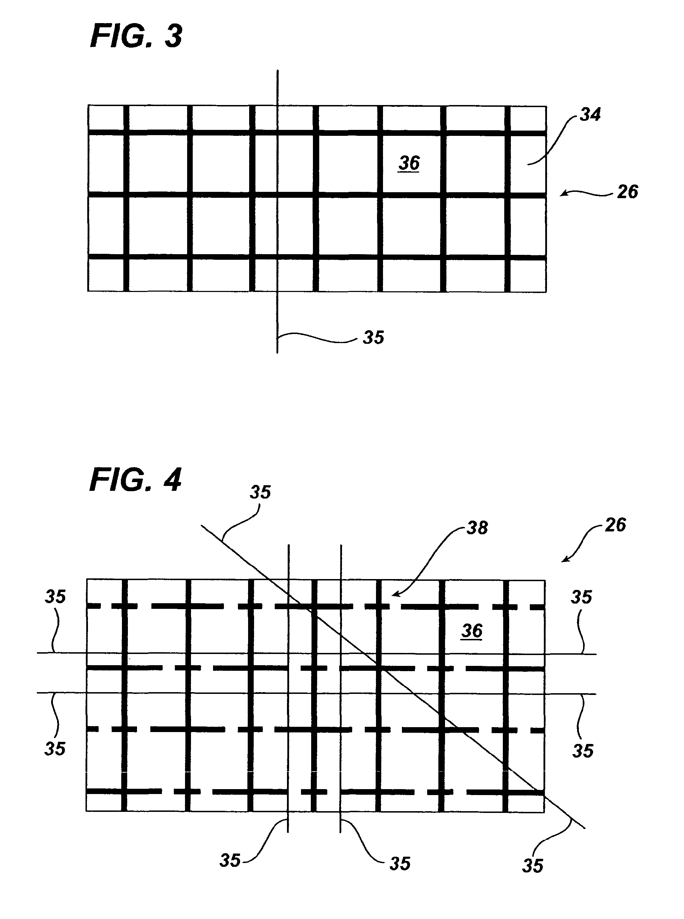

Turning now also to FIG. 3, a pattern (in this embodiment - an orthogonal, continuous grid) of ridges 34 is formed on a lower surface 36 of the mat 26. To decrease the tendency of the mat 26 to roll the ridges 34 are formed into a pattern which leaves no fold-lines 35 extending from one side of the mat 26 to the other without crossing the ridges 34. Prior mats with ridges allowed fold lines to form unimpeded by any ridges. Of course, these prior ridges were intended to elevate the mat above the tray, not limit folding.

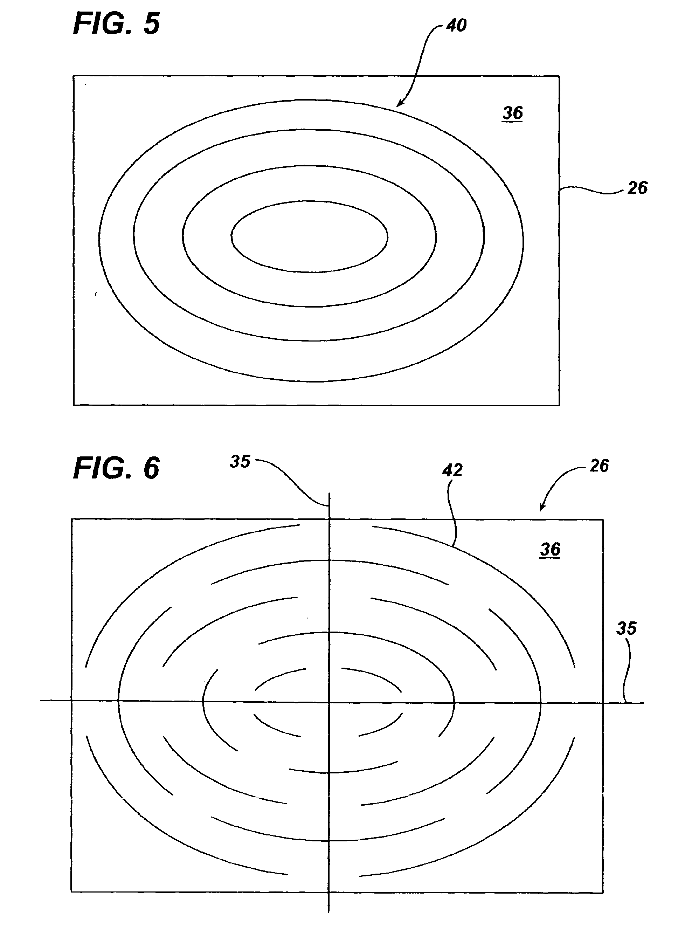

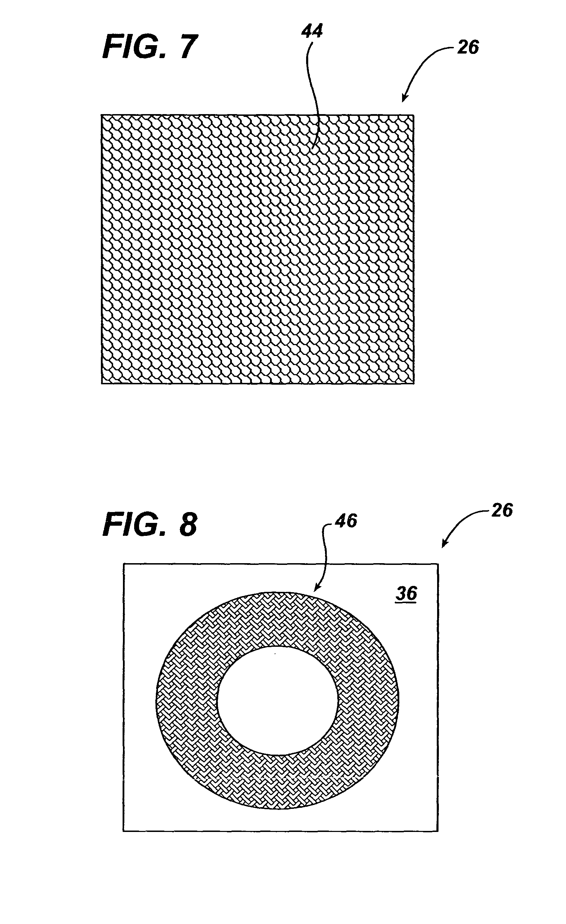

Turning also to FIGS. 4 to 8, FIG. 4 illustrates a different pattern of ridges 38, a discontinuous, orthogonal pattern. FIG. 5 illustrates a further pattern of ridges 40, continuous, concentric ovals. FIG. 6 illustrates one further pattern of ridges 42, discontinuous, concentric ovals. FIG. 7 illustrates a an interlocking pattern of ridges 44 and FIG. 8 illustrates one further pattern of ridges 46 arranged in an oval on the mat 26. It will be understood that many patterns of ridges may be employed in keeping with the spirit of the invention.

The ridges in each of these embodiments eliminate fold lines, at least through a central portion of the mat 26. Preferably, the ridges extend over the entire mat bottom surface 36 to prevent any unimpeded fold lines 35. The ridges are preferably formed with a height of between 0.5 mm to 10.0 mm, more preferably in the range of 0.75 mm to 5.0 mm and most preferably in the range of 1.0 mm to 4.0 mm.

Prior mats with ridges attempted to minimize contact between the mat and the tray bottom to prevent trapped liquid. However, this exacerbates the problem of mat slippage as insufficient surface area contacts the tray to provide good frictional adhesion between the mat and the tray. A more densely packed pattern of ridges provides more surface to contact and adhere to the tray.

The invention now being fully described, it will be apparent to one of ordinary skill in the art that many modifications and changes can be made thereto without departing from the spirit or scope of the invention is defined in the following claims.

## Claims

1. A sterilization container system comprising:
an enclosure defining an interior volume;
an upwardly facing surface in the interior volume;
a flexible elastomeric mat having a downwardly facing surface resting upon the upwardly facing surface; and
wherein the downwardly facing surface is provided with a pattern of ridges and wherein the pattern is such that no fold line can traverse the mat from one side to the other without intersecting at least one ridge.

2. A sterilization container system according to claim 1 wherein the pattern is such that no fold line can traverse the mat from one side to the other without intersecting a plurality of the ridges.

3. A sterilization container system wherein the ridges have a height of 0.5 mm to 10.0 mm.

4. A sterilization container system according to claim 3 wherein the ridges have a height of 0.75 mm to 5.0 mm.

5. A sterilization container system according to claim 4 wherein the ridges have a height of 1.0 mm to 4.0 mm.

6. A sterilization container according to claim 1 wherein the flexible elastomeric mat is formed of silicone.

7. A sterilization container according to claim 6 wherein the silicone has a hardness of less than 90A on the Shore A Scale.

8. A sterilization container according to claim 6 wherein the pattern extends substantially across a dimension of the lower surface.

9. A sterilization container according to claim 1 wherein the mat further comprises a plurality of upwardly projecting members.

10. A sterilization container according to claim 1 wherein the mat further comprises a plurality of apertures therethrough.

11. A sterilization container according to claim 1 wherein the pattern is continuous.

12. A sterilization container according to claim 1 wherein the pattern is discontinuous.

13. A sterilization container according to claim 1 wherein the pattern comprises concentric shapes.

14. A method of sterilizing an item comprises the steps of:
placing a flexible, elastomeric mat into an enclosure having an interior volume;
placing the item onto the mat; and
levitating the mat above a bottom surface of the enclosure via a pattern of ridges on a lower surface of the mat, the pattern being such that no fold line can traverse the mat from one side to the other without intersecting at least one ridge.
